# EUROPÄISCHE PATENTANMELDUNG

(11) **EP 2 028 178 A1**
(43) Veröffentlichungstag der Anmeldung: **25.02.2009**
(21) Anmeldenummer: 07016422.3
(22) Anmeldetag: 22.08.2007
(51) Int. Cl.: C07C 263/10, C07C 265/14, C01B 31/28

(54) **Herstellung von Isocyanaten mit niedrigen Chlorgehalten**

(71) Anmelder: Bayer MaterialScience AG, 51368 Leverkusen (DE)
(72) Erfinder: Stutz, Herbert, 41541 Dormagen (DE); Diehl, Frank, 42799 Leichlingen (DE); Bruns, Rainer, 51373 Leverkusen (DE); Schwarz, Alexander, 51371 Leverkusen (DE)

(57) **Zusammenfassung**

Die vorliegende Erfindung betrifft ein neuartiges Verfahren zur Herstellung von Isocyanaten, welche sich durch einen besonders niedrigen Gehalt an Verbindungen auszeichnen, welche Chlor in gegebenenfalls hydrolysierbarer Form enthalten.

## Beschreibung

Die vorliegende Erfindung betrifft ein neuartiges Verfahren zur Herstellung von Isocyanaten, welche sich durch einen besonders niedrigen Gehalt an Verbindungen auszeichnen, welche Chlor in gegebenenfalls hydrolysierbarer Form enthalten.

Die Phosgenierung von aliphatischen oder aromatischen Aminen zur Herstellung von Isocyanaten kann besonders vorteilhaft in der Gasphase durchgeführt werden. Solche Verfahren sind grundsätzlich im Stand der Technik seit langem bekannt und industriell mittlerweile etabliert (EP-B 289 840).

Zur Phosgenierung im industriellen Maßstab wird üblicherweise in einem Phosgenerzeuger an einem Katalysator aus Chlor und Kohlenmonoxid hergestelltes Phosgen eingesetzt. Das Phosgen aus dem Erzeuger wird vor der Zuführung in die Phosgenierung zunächst noch einer Reinigungsstufe zugeführt und dort, vorzugsweise durch Auskondensieren von Phosgen oder durch Destillation in einen Abgasstrom und einen Phosgenstrom getrennt. Der Phosgenstrom wird dann vor der eigentlichen Umsetzung mit dem Amin noch meist mit zurückgeführtem Phosgen vermischt (WO 2004/037718).

Dieses dem Stand der Technik nach eingesetzte Phosgen besitzt aufgrund der Nachbehandlungsschritte Restgasgehalte an CO von weniger als 0,05 Gew.-%

Nach der Umsetzung von Amin und Phosgen wird das resultierende Isocyanat in bekannter Weise destillativ von leichtsiedenden und hochsiedenden Nebenprodukten gereinigt. Vielfach problematisch ist es, wenn die gereinigten Isocyanate gefärbt sind oder es bei anschließenden Modifizierungsschritten wie beispielsweise der Prepolymerisierung, Biuretisierung oder Trimerisierung zu unerwünschten Nebenreaktionen kommt, die wiederum letztendlich die Färbung der dabei erhaltenen Polyisocyanate negativ beeinflusst.

Solche Nebenreaktionen werden oftmals durch sehr geringe Konzentrationen an Verbindungen ausgelöst, welche chlorhaltig sind bzw. Chlor in hydrolysierbarer Form umfassen. Grundsätzlich ist eine Abtrennung von solchen Verbindungen, die zum Gehalt an hydrolysierbarem Chlor (HC-Verbindungen bzw. -Gehalt) oder zum Gesamtchlorgehalt der Isocyanate beitragen, möglich, jedoch produktionstechnisch unerwünscht, da ein solcher zusätzlicher Aufreinigungsschritt die Produktionskosten durch Mehrverbrauch an Energie bzw. Ausbeuteverlust durch thermische Belastung erhöht.

Aufgabe war es daher ein Verfahren bereitzustellen, welches verglichen mit denjenigen des Standes der Technik ohne weitere Aufreinigungsschritte zu Produkten mit signifikant reduzierten HC- und Gesamtchlorgehalten führt.

Überraschenderweise wurde nun gefunden, dass eine Senkung der Gehalte an Verbindungen, welche zum HC-Gehalt sowie zum Gesamtchlorgehalt beitragen, dadurch erzielt werden kann, wenn das der Aminphosgenierung zugeführte Phosgen im Mittel einen Gehalt an Kohlenmonoxid von mehr als 0,5 Gew.-% aufweist.

Gegenstand der vorliegenden Erfindung ist daher die Verwendung von Phosgen mit einem mittleren Kohlenmonoxidgehalt von 0,5 Gew.-% oder mehr zur Phosgenierung von Aminen.

Gegenstand der Erfindung ist ferner ein Verfahren zur Herstellung von Isocyanaten durch Umsetzung der korrespondierenden Amine mit Phosgen gegebenenfalls in Anwesenheit eines Inertmediums, wobei der der Phosgenierung zugeführte Phosgenstrom im Mittel einen CO-Gehalt von 0,5 Gew.-% oder mehr aufweist.

Bevorzugt weist der Phosgenstrom einen CO-Gehalt von mehr als 1 Gew.-%, besonders bevorzugt von mehr als 2 Gew.-% auf. Der CO-Gehalt im Phosgen liegt jedoch bevorzugt unterhalb von 8 Gew.-%, ganz besonders bevorzugt unterhalb von 4 Gew.-%.

Zur Bereitstellung des CO-Gehalts im Phosgen ist es unerheblich, ob das CO dem Phosgen separat zugeführt wird oder ob ein aus der Phosgenherstellung anfallender Phosgenstrom als Phosgenquelle für die erfindungsgemäße Phosgenierung verwendet wird, der bereits CO im anspruchsgemäßen Bereich enthält.

Bevorzugt wird das aus dem Phosgenerzeuger unmittelbar erhaltene Gemisch aus CO und Phosgen ohne weitere Aufarbeitung und Abreicherung von CO der Phosgenierung zugeführt.

Bevorzugtes Verfahren zur Phosgenerzeugung ist dabei die in EP 1 640 341 beschriebene Herstellung von Phosgen aus Chlor und Kohlenmonoxid in Gegenwart eines Aktivkohle-Katalysators in einem Rohrbündelreaktor unter effizienter Kühlung mit Gastemperaturen von unter 100°C. Das bevorzugte Verfahren zeichnet sich dadurch aus, dass die Abfuhr der Reaktionswärme durch Verdampfungskühlung mit Wasser bei vermindertem Druck unterhalb des atmosphärischen Drucks von 1 bar absolut erfolgt. Der Kühlmittelkreislauf ist ein geschlossener Kreislauf in dem Wasser verdampft, abgeleitet, an anderer Stelle kondensiert und anschließend zur erneuten Verdampfung in den Kühlmittelraum zurückgeleitet wird. Der Kühlmittelraum enthält dabei bevorzugt zu jeder Zeit flüssiges Wasser im Siedezustand. Die Einsatzströme CO und Chlor werden in dem bevorzugten Verfahren zur Erzielung eines niedrigen Chlorgehaltes im Phosgen bevorzugt in einem molaren Überschuss an CO von 2 bis 20 %, besonders bevorzugt 5 bis 15% eingesetzt.

Bevorzugt weist der der Reaktionszone zugeführte Phosgenstrom einen HCl-Gehalt von weniger als 15 Gew.-%, besonders bevorzugt von 0,1 bis 10 Gew.-%, ganz besonders bevorzugt 2 bis 8 Gew.-% auf.

Das erfindungsgemäße Verfahren wird bevorzugt einstufig durchgeführt. Darunter ist zu verstehen, dass die Vermischung und Umsetzung der Edukte zum Produkt in einer Reaktionszone erfolgt. Nach Verlassen der Produkte aus der Reaktionszone ist eine bevorzugt vollständige Umsetzung der zugeführten Aminogruppen mit Phosgen erfolgt. Dies ist insbesondere deshalb anzustreben, weil ansonsten noch nicht umgesetzte Aminogruppen zur Hydrochlorid- oder Harnstoffbildung führen können, was die Gesamtausbeute an Isocyanat verringert und aufgrund der Bildung von Ablagerungen die Reaktorstandzeit und/oder die Standzeit der nachfolgenden Aufarbeitungsapparatur verringert wird.

Ebenfalls bevorzugt ist ein kontinuierlicher Betrieb des erfindungsgemäßen Verfahrens.

Für das erfindungsgemäße Verfahren können sämtliche aminofunktionellen Verbindungen mit wenigstens einer, bevorzugt 1 bis 3 primären Aminogruppen eingesetzt werden, sofern sie in die Dampfform überführt werden können Es ist dabei unerheblich ob die Amine aliphatischer, cycloalophatischer, araliphatischer oder aromatischer Natur sind.

Vorgenannte aminofunktionellen Verbindungen weisen üblicherweise bis zu 22 Kohlenstoffatome auf, wobei, falls mehrere Aminogruppen im Molekül vorliegen, diese durch wenigstens 2 Kohlenstoffatome von einander getrennt sind.

Bevorzugt werden Amine der vorgenannten Art eingesetzt, die ohne nennenswerte Zersetzung in die Gasphase überführt werden können.

Besonders geeignet sind zu diesem Zweck Diamine auf Basis von aliphatischen oder cycloaliphatischen Kohlenwasserstoffen mit 2 bis 18 Kohlenstoffatomen.

Beispiele hierfür sind 1,4-Diaminobutan, 1,6-Diaminohexan, 1,8-Diaminooctan, 1,10-Diaminodecan, 1,6-Diamino-2,4,4-trimethyl-hexan und 1,6-Diamino-2,2,4-trimethyl-hexan, 1-Amino-3,3,5-trimethyl-5-aminomethylcyclohexan (IPDA), 2,4-, oder 2,6-Diamino-1-methylcyclohexan und 4,4'- und/oder 4,2'-Diaminodicyclohexyl-methan. Besonders bevorzugt sind 1,6-Diamino-hexan, 1-Amino-3,3,5-trimethyl-5-(aminomethyl)cyclohexan, und 4,4'-und/oder 4,2'-Di-(aminocyclohexyl)-methan.

Ebenfalls als Ausgangsmaterialien geeignet sind beliebige (cyclo)aliphatische Triamine mit bis zu 22 Kohlenstoffatomen, soweit sie unter den Temperaturbedingungen des erfindungsgemäßen Verfahrens stabil sind und in die Dampfform überführt werden können. Beispielsweise können dies Triaminocyclohexan, Tris(aminomethyl)-cyclohexan, Triamino-methylcyclohexan sein. Ebenfalls geeignet sind 1,8-Diamino-4-(aminomethyl)octan, 1,6,11 -Undecantriamin, 1,7-Diamino-4-(3-aminopropyl)heptan, 1,6-Diamino-3-(aminomethyl)-hexan oder 1,3,5-Tris(aminomethyl)-cyclohexan.

Ebenfalls können für das erfindungsgemäße Verfahren aromatische Amine verwendet werden, die bevorzugt ohne nennenswerte Zersetzung in die Gasphase überführt werden können. Beispiele für bevorzugte aromatische Amine sind Toluylendiamin (TDA), als 2,4- oder 2,6-Isomer oder als Gemisch davon, Diaminobenzol, 2,6-Xylidin, Napthylendiamin (NDA) und 2,4'- oder 4,4'-Methylen(di-phenylamin) (MDA) oder Isomerengemische davon. Bevorzugt sind 2,4- und/oder 2,6-TDA.

Die Edukte Amin und Phosgen können jeweils auch zusammen mit einem Inertmedium in den Reaktionsraum eindosiert werden. Bei dem Inertmedium handelt es sich um ein Medium, das bei der Reaktionstemperatur gasförmig im Reaktionsraum vorliegt und nicht mit den im Reaktionsverlauf auftretenden Verbindungen reagiert. Das Inertmedium wird im Allgemeinen vor der Umsetzung mit Amin und/oder Phosgen vermischt, kann aber auch getrennt von den Eduktströmen zudosiert werden. Beispielsweise können Stickstoff, Edelgase, wie Helium oder Argon, oder Aromaten, wie Chlorbenzol, Dichlorbenzol, Xylol oder Kohlenstoffdioxid, verwendet werden. Bevorzugt wird Stickstoff und/oder Chlorbenzol und / oder Dichlorbenzol als Inertmedium verwendet.

Im Allgemeinen wird das Inertmedium in einer Menge eingesetzt, so dass das Verhältnis von Gasvolumen des Inertmedium zu Gasvolumen des Amins bzw. Phosgens bei 0,001 bis 5, bevorzugt 0,01 bis 3, besonders bevorzugt 0,1 bis 1 liegt. Bevorzugt wird das Inertmedium zusammen mit den Aminen in den Reaktionsraum eingeführt.

Das erfindungsgemäße Verfahren wird bevorzugt so durchgeführt, dass die Edukte Amin und Phosgen als auch das in der Reaktionszone entstehende Isocyanat unter den Reaktionsbedingungen in gasförmigem Zustand sind, dass heißt, dass eine Bildung von Flüssigkeitströpfen bevorzugt ausgeschlossen ist.

Zur Bereitstellung der vorgenannten Reaktionsbedingungen betragen die Temperaturen in der Reaktionszone bevorzugt mehr als 200°C, besonders bevorzugt mehr als 260°C, ganz besonders mehr als 280°C. Die Temperaturobergrenze liegt dabei bevorzugt unterhalb 570°C, besonders bevorzugt unterhalb von 500°C.

Die Umsetzung von Phosgen mit Amin in der jeweiligen Reaktionszone erfolgt bei Absolutdrücken von mehr als 0,1 bar bis weniger als 20 bar, bevorzugt 0,5 bar bis 10 bar, besonders bevorzugt 0,7 bar bis 5 bar, ganz besonders bevorzugt 0,8 bis 3 bar.

Im allgemeinen ist der Druck in den Zuleitungen in die Reaktionszone höher, als der vorstehend angegebene Druck in der Reaktionszone selber. Bevorzugt ist der Druck in den Zuleitungen um 20 bis 2000 mbar, besonders bevorzugt von 30 bis 1000 mbar höher als in der Reaktionszone selbst.

Im allgemeinen ist der Druck in der sich an die eigentliche Reaktionszone anschließenden Bereichen des Verfahrens niedriger als in den Reaktionszonen selbst. Bevorzugt ist der Druck dort um 10 bis 500 mbar, besonders bevorzugt 30 bis 150 mbar, niedriger als in der Reaktionszone.

Bevorzugt werden die Edukte mit einer Strömungsgeschwindigkeit von je 1 bis 100 m/s, besonders bevorzugt von 2 bis 50 m/s in und durch die Reaktionszone geleitet.

Die Strömungsgeschwindigkeiten der beiden Edukte werden innerhalb der vorgenannten Bereiche bevorzugt so eingestellt, dass in der Reaktionszone eine mittlere Kontaktzeit des Umsetzungsgemisches aus Aminen und Phosgen im allgemeinen von 0,01 Sekunden bis weniger als 15 Sekunden, bevorzugt von mehr als 0,04 Sekunden bis weniger als 10 Sekunden, besonders bevorzugt von mehr als 0,08 Sekunden bis weniger als 5 Sekunden erreicht wird. Unter mittlerer Kontaktzeit wird die Zeitspanne vom Beginn der Vermischung der Edukte bis zum Verlassen des Reaktionsraumes in die Aufarbeitungsstufe verstanden. In einer bevorzugten Ausführungsform ist die Strömung im erfindungsgemäßen Verfahren durch eine Bodenstein-Zahl von mehr als 10, bevorzugt mehr als 100 und besonders bevorzugt von mehr als 250 charakterisiert.

Vorteilhafterweise werden die Abmessungen des Reaktionsraums und die Strömungsgeschwindigkeiten dabei so gewählt, dass eine turbulente Strömung, d.h. eine Strömung mit einer Reynolds-Zahl von mindestens 2300, bevorzugt mindestens 2700, für das Reaktionsgemisch vorliegt, wobei die Reynolds-Zahl mit dem hydraulischen Durchmesser des Reaktionsraumes gebildet wird.

Durch die turbulente Strömung werden eine enge Verweilzeit mit geringer Standardabweichung von unterhalb 10 %, bevorzugt unterhalb 6 % erreicht.

Bevorzugt besitzt die Reaktionszone keine beweglichen Einbauten.

Die Reaktionszone kann über seine Außenfläche temperiert werden. Um Produktionsanlagen mit hoher Anlagenkapazität zu bauen, können mehrere Reaktorrohre parallel geschaltet werden. Die Umsetzung kann aber auch bevorzugt adiabat erfolgen. Das bedeutet, dass über die Außenfläche des Reaktionsvolumens nicht mit technischen Maßnahmen Heiz- oder Kühlenergieströme fließen. Bevorzugt findet die Umsetzung adiabat statt.

Nachdem das Reaktionsgemisch in der Reaktionszone umgesetzt wurde, ist eine rasche Abkühlung der Reaktionsgase nach der Phosgenierungsreaktion auf Temperaturen unterhalb 180°C notwendig, um die Bildung unerwünschter Nebenprodukte durch den thermischen Zerfall von Di-/Triisocyanat oder durch eine Weiterreaktion durch Polymerisation zu vermeiden, da die gebildeten Di-/Triisocyanate bei den Reaktionstemperaturen von 300 bis 570°C thermisch nicht stabil sind. Die Abkühlung auf Temperaturen von 100 bis 180 °C erfolgt in einer ein- oder mehrstufigen Wäsche (Quenche mit Waschkolonne) mit einem inerten Lösungsmittel, wie in EP-A1 1403248, Sp. 2, Z. 39 - Sp. 3, Z. 18 beschrieben.

Als Lösungsmittel sind bevorzugt Kohlenwasserstoffe, die gegebenenfalls mit Halogenatomen substituiert sind, geeignet, wie beispielsweise Chlorbenzol, Dichlorbenzol, und Toluol. Als Lösungsmittel wird besonders bevorzugt Monochlorbenzol und/oder Dichlorbenzol eingesetzt. Als Lösungsmittel kann auch das Isocyanat oder eine Lösung des hergestellten Isocyanates in den genannten Lösungsmitteln, die auch über einen Wärmetauscher zur Energieabfuhr im Kreis gefahren werden kann, eingesetzt werden. Bei der Wäsche wird das Isocyanat selektiv in die Waschlösung übergeführt. Aus dem verbleibenden isocyanatfreien Gas (überschüssiges Phosgen, Chlorwasserstoff , gegebenenfalls das Inertmedium und Lösungsmittel aus der Wäsche) wird das Lösungsmittel durch partielle Kondensation und anschließend das Phosgen zurückgewonnen, z.B. mittels Absorption in Monochlorbenzol und/oder Dichlorbenzol und der Chlorwasserstoff gegebenenfalls nach Reinigung gemäß Stand der Technik als Rohstoff weiterverwendet. Die in der Quenche und Waschkolonne erhaltene konzentrierte Isocyanatlösung hat eine eine Isocyanatkonzentration von mindestens 20 Gew.-%, bevorzugt von mindestens 25 Gew.-%. Die in der Quenche und Waschkolonne erhaltene konzentrierte Isocyanatlösung wird bevorzugt mittels Rektifikation von physikalisch (gelöstem) und chemisch gebundenem Chlorwasserstoff und Phosgen befreit und in weiteren Destillationsstufen in reines Lösungsmittel, leichtsiedende Nebenprodukte, reines Di-oder Triisocyanat und Schwersieder aufgetrennt. Bevorzugt wird das Isocyanat verwendet.

Die nach dem erfindungsgemäßen Verfahren erhältlichen (cyclo)aliphatischen Isocyanate besitzen Gehalte an Verbindungen mit hydrolysierbarem Chlor von bevorzugt weniger als 200 ppm, besonders bevorzugt weniger als 80 ppm.

Die nach dem erfindungsgemässen Verfahren erhältlichen aromatischen Isocyanate besitzen Gehalte an Verbindungen mit hydrolysierbarem Chlor von bevorzugt weniger als 100 ppm, besonders bevorzugt weniger als 40 ppm.

Der Gesamtchlorgehalt liegt bei den (cyclo)aliphatischen und bei den aromatischen Isocyanaten bevorzugt unterhalb von 800 ppm, besonders bevorzugt unterhalb von 500 ppm.

Die Bestimmung des Gehalts an hydrolysierbarem Chlor in Isocyanaten im Arbeitsbereich w(C1) > 5 mg/kg erfolgt durch Urethanisierung, Hydrolyse und potentiometrische Tritration mit Silbernitrat an einer Silber/Silberchlorid-Einstabmesskette.

Zur Bestimmung des Gehalts an hydrolysierbarem Chlor wird die Isocyanatprobe mit Methanol versetzt und 10 min unter Rückfluß urethanisiert. Anschließend wird die Mischung nach Verdünnen mit Wasser durch Kochen unter Rückfluß hydrolysiert. Das hierbei gebildete ionogene Chlor wird nach Ansäuern mit Salpetersäure und Aufstockung mit einer bekannten Masse Natriumchlorid argentometrisch mit einer Silbernitratmasslösung titriert. Die Titration wird mit inkrementeller Reagenzdosierung und automatischer Equivalenzpunkt-Auswertung driftkontrolliert (Gleichgewichtstitration) durchgeführt. Aus der Einwaage der Isocyanatprobe und dem Verbrauch an Silbernitratmasslösung wird der Gehalt an hydrolysierbarem Chlor unter Berücksichtiguaang der Aufstockung errechnet.

Die nach dem erfindungsgemässen erhältlichen Isocyanate lassen sich besonders vorteilhaft bei der Herstellung von Polyurethanlacken sowie Kleb- und Dichtstoffen und flexiblen sowie harten Schaumstoffen einsetzen. Dazu werden sie bevorzugt zunächst zu Prepolymeren, Uretdionen, Isocyanuraten, Iminooxadiazindionen, Biureten oder Allophanaten umgesetzt sowie gegebenenfalls mit in der Technik an sich üblichen Methoden blockiert.

### Beispiele:

### Beispiel 1 (nicht erfindungsgemäßes Beispiel):

Durch Vermischung in einer Glattstrahldüse wurden einem Rohrreaktor mit nachgeschalteter Isocyanatkondensationsstufe und einer nachfolgenden destillativen Isocyanataufarbeitung als Eduktstrom A ein Isophorondiamin-Inertgas-Gemisch und als Eduktstrom B Phosgen, das durch Verdampfen einer Lösung von Phosgen (wiedergewonnener Überschuß) in Monochlorbenzol und durch Verdampfen von kondensiertem Phosgen aus der Phosgenerzeugung gewonnen wurde, in einer Menge von 160% der Theorie kontinuierlich zugeleitet. Der gasförmige Phosgenstrom enthielt 2,9 Gew % Chlorwasserstoff und > 0,05 Gew % Kohlenmonoxid. Die Temperaturen der beiden Eduktströme betrugen 300°C. Der Druck in dem Rohrreaktor lag geringfügig oberhalb des Atmosphärendruckes bei 1300 mbar.

Die Geschwindigkeit der Komponente A in der Glattstrahldüse betrug ca. 80 m/s, die der Komponente B vor der Vermischung ca. 10 m/s. Durch die adiabate Reaktionsführung stieg die Temperatur im Reaktor auf ca. 420°C an.

Das Reaktionsprodukt Isophorondiisocyanat (IPDI) wurde nach Verlassen des Reaktors kondensiert, vom Nebenprodukt Chlorwasserstoff, den Inerten und dem überschüssigen Phosgen getrennt und anschließend in der Destillationssequenz gereinigt. Das erhaltene IPDI hatte einen Gehalt an hydrolysierbarem Chlor von 140 ppm und einen Gesamtchlor-Gehalt von 190 ppm.

Das so erhaltene IPDI wurde zu dem Trimerisat, einem trifunktionellen Polyisocyanat mit sehr geringem Dampfdruck weiterverarbeitet, das in Lacksystemen Anwendung findet. Das Trimerisat, gelöst zu 70% in einem handelsüblichen Lacklösemittel war nahezu wasserklar mit einer Hazen-Farbzahl von 50 Apha

### Beispiel 2 (erfindungsgemäß):

Beispiel 1 wurde unter den gleichen Bedingungen wiederholt, wobei der Eduktstrom B Phosgen wieder 160% der Theorie betrug, zu 60% durch Verdampfen einer Lösung von zurückgeführtem Phosgen gewonnen wurde und das zur Umsetzung erforderliche Phosgen direkt aus der Phosgenerzeugung gasförmig inklusive der Inerten und des überschüssigen Kohlenmonoxids zugeführt wurde. Der gasförmige Phosgenstrom vor Eintritt in den Reaktor enthielt 3,1 Gew % Chlorwasserstoff und 2,3 Gew % Kohlenmonoxid.

Das aus der Destillationssequenz erhaltene IPDI hatte einen Gehalt an hydrolysierbarem Chlor von 80 ppm und einen Gesamtchlor-Gehalt von 100 ppm.

Das so erhaltene IPDI wurde ebenfalls zu dem Trimerisat weiterverarbeitet. Das Trimerisat, gelöst zu 70% in einem handelsüblichen Lacklösemittel war nahezu wasserklar mit einer Hazen-Farbzahl von 30 Apha.

### Beispiel 3 (nicht erfindungsgemäßes Beispiel):

Durch Vermischung in einer Glattstrahldüse werden einem Rohrreaktor mit nachgeschalteter Isocyanatkondensationsstufe und dieser nachfolgenden destillativen Isocyanataufarbeitung als Eduktstrom A ein Hexamethylendiamin-Inertgas-Gemisch und als Eduktstrom B Phosgen, das durch Verdampfen einer Lösung von Phosgen ( wiedergewonnener Überschuß und kondensiertes Phosgen aus der Phosgenerzeugung ) in Monochlorbenzol gewonnen wird, in einer Menge von 220% der Theorie kontinuierlich zugeleitet. Der Eduktstrom B, Phosgen, enthielt 6,9 Gew% Chlorwasserstoff und > 0,05 Gew% Kohlenmonoxid. Die Temperaturen der beiden Eduktströme betragen 300°C. Der Druck in dem Rohrreaktor liegt geringfügig oberhalb des Atmosphärendruckes bei 1400 mbar.

Die Geschwindigkeit der Komponete A in der Glattstrahldüse betrug ca. 50 m/s, die der Komponente B vor der Vermischung ca. 10 m/s. Infolge der adiabaten Reaktionsführung erhöhte sich die Temperatur im Reaktor auf ca. 450°C.

Das Reaktionsprodukt Hexamethylendiisocyanat (HDI) wurde nach Verlassen des Reaktors kondensiert, vom Nebenprodukt Chlorwasserstoff, den Inerten und dem überschüssigen Phosgen getrennt und anschließend in der Destillationssequenz gereinigt. Das erhaltene HDI hatte einen Gehalt an hydrolysierbarem Chlor von 50 ppm und einen Gesamtchlor-Gehalt von 430 ppm.

Das so erhaltene HDI wurde zu dem Trimerisat, einem trifunktionellen Polyisocyanat mit sehr geringem Dampfdruck weiterverarbeitet, das in Lacksystemen Anwendung findet. Das Trimerisat war nahezu wasserklar mit einer Hazen-Farbzahl von 80 Apha

### Beispiel 4 (erfindungsgemäß):

Beispiel 3 wurde unter den gleichen Bedingungen wiederholt, wobei der Eduktstrom B Phosgen wieder 220% der Theorie betrug, zu 120% durch Verdampfen einer Lösung von zurückgeführtem Phosgen gewonnen wurde und das zur Umsetzung erforderliche Phosgen direkt aus der Phosgenerzeugung inklusive der Inerten und des Kohlenmonoxids zugeführt wurde. Der Eduktstrom B, Phosgen, enthielt 6,3 % Chlorwasserstoff und 3,4 Gew% Kohlenmonoxid.

Das aus der Destillationssequenz erhaltene HDI hatte einen Gehalt an hydrolysierbarem Chlor von 20 ppm und einen Gesamtchlor-Gehalt von 190 ppm.

Das so erhaltene HDI wurde ebenfalls zu dem Trimerisat weiterverarbeitet. Das Trimerisat war nahezu wasserklar mit einer Hazen-Farbzahl von 20 Apha

### Beispiel 5 (erfindungsgemäß):

Durch Vermischung in einer Düse werden einem Rohrreaktor mit nachgeschalteter Isocyanatkondensationsstufe und dieser nachfolgenden destillativen Isocyanataufarbeitung als Eduktstrom A ein Gemisch bestehend aus gasförmigen 2,4- und 2,6-Toluylendiamin sowie Inertgas und als Eduktstrom B Phosgen, das zu 75% durch Verdampfen einer Lösung von Phosgen (wiedergewonnener Überschuß) und das zur Umsetzung erforderliche Phosgen direkt aus der Phosgenerzeugung gasförmig inklusive der Inerten und des überschüssigen Kohlenmonoxids zugeführt. Der gasförmige Phosgenstrom aus der Phosgenerzeugung enthielt 5,0 % Kohlenmonoxid.

Der Druck in dem Rohrreaktor liegt geringfügig oberhalb des Atmosphärendruckes bei 1500 mbar. Infolge der adiabaten Reaktionsführung erhöhte sich die Temperatur im Reaktor auf ca. 450°C.

Das Reaktionsprodukt als Gemisch bestehend aus 2,4- und 2,6-Toluylendiisocyanat (TDI) wurde nach Verlassen des Reaktors kondensiert, vom Nebenprodukt Chlorwasserstoff, den Inerten und dem überschüssigen Phosgen getrennt und anschließend in der Destillätionssequenz gereinigt. Das erhaltene TDI hatte einen Gehalt an hydrolysierbarem Chlor von 30 ppm und einen Gesamtchlor-Gehalt von 80 ppm mit einer Hazen-Farbzahl von 20 Apha.

## Patentansprüche

1. Verfahren zur Herstellung von Isocyanaten durch Umsetzung der korrespondierenden Amine mit Phosgen gegebenenfalls in Anwesenheit eines Inertmediums, wobei der der Phosgenierung zugeführte Phosgenstrom im Mittel einen CO-Gehalt von 0,5 Gew.-% oder mehr aufweist.

2. Verfahren gemäß Anspruch 1, **dadurch gekennzeichnet, dass** die Phosgenierung der Amine derart geführt wird, dass eine Bildung von Flüssigkeitströpfen in der Reaktionszone ausgeschlossen ist.

3. Verfahren gemäß Anspruch 1 oder 2, **dadurch gekennzeichnet, dass** der zugeführte Phosgenstrom im Mittel einen CO-Gehalt von 1 Gew.-% oder mehr aufweist.

4. Verfahren gemäß einem der Ansprüche 1 bis 3, **dadurch gekennzeichnet, dass** der der Reaktionszone zugeführte Phosgenstrom im Mittel einen CO-Gehalt von weniger als 8 Gew.-% aufweist.

5. Verfahren gemäß einem der Ansprüche 1 bis 4, **dadurch gekennzeichnet, dass** der der Reaktionszone zugeführte Phosgenstrom einen HCl-Gehalt von 0,1 bis 10 Gew.-% aufweist.

6. Verfahren gemäß einem der Ansprüche 1 bis 5, **dadurch gekennzeichnet, dass** es sich bei dem Inertmedium um Stickstoff und/oder Chlorbenzol und/oder Dichlorbenzol handelt.

7. Verfahren gemäß einem der Ansprüche 1 bis 6, **dadurch gekennzeichnet, dass** die Umsetzung in der Reaktionszone bei mehr als 200°C aber weniger als < 570°C und Absolutdrücken von 0,8 bis 3 bar durchgeführt wird.

8. Verfahren gemäß Anspruch 7, wobei der Druck in den Amin bzw. Phosgenzuleitungen um 20 bis 2000 mbar oberhalb und der Druck in den der Reaktionszone nachgeschalteten Zonen um 10 bis 500 mbar unterhalb des Drucks in der Reaktionszone liegt.

9. Verfahren gemäß einem der Ansprüche 1 bis 8, **dadurch gekennzeichnet, dass** die Edukte mit einer Strömungsgeschwindigkeit von je 1 bis 100 m/s in und durch die Reaktionszone geleitet werden und die mittlere Kontaktzeit des Umsetzungsgemisches aus Aminen und Phosgen im allgemeinen von 0,01 Sekunden bis weniger als 15 Sekunden beträgt.

10. Verfahren gemäß einem der Ansprüche 1 bis 9, **dadurch gekennzeichnet, dass** das Verfahren adiabat geführt wird.

11. Verfahren gemäß einem der Ansprüche 1 bis 10, **dadurch gekennzeichnet, dass** die so erhältlichen Produkte im Falle aliphatischer oder cycloaliphatischer Isocyanate Gehalte an Verbindungen mit hydrolysierbarem Chlor von weniger als 200 ppm und im Falle von aromatischen aromatischen Isocyanaten von weniger als 100 ppm aufweisen.

12. Verfahren gemäß einem der Ansprüche 1 bis 11, **dadurch gekennzeichnet, dass** das zugeführte Phosgen unmittelbar ohne weitere Reinigungsschritte aus der Phosgenerzeugung als Frisch-Phosgen in die Phosgenierungsreaktion eingespeist wird.

13. Verfahren gemäß Anspruch 12, **dadurch gekennzeichnet, dass** bei der Phosgenherstellung ein molarer Überschuss an CO zu Chlor von 5 bis 20 % vorliegt.

14. Verfahren gemäß Anspruch 12 oder 13, **dadurch gekennzeichnet, dass** das Frisch-Phosgen vor Einspeisung in die Phosgenierung mit Phosgen angereichert wird, welches nach der Isocyanatherstellung zurückgewonnen und recycliert wurde.

15. Verwendung von Phosgen mit einem mittleren Kohlenmonoxidgehalt von 0,5 Gew.-% oder mehr zur Phosgenierung von Aminen.
